# EUROPEAN PATENT APPLICATION

(11) **EP 1 364 593 A2**
(43) Date of publication of application: **26.11.2003**
(21) Application number: 03009477.5
(22) Date of filing: 26.04.2003
(51) Int. Cl.: A45D 29/16, A61B 17/54

(54) **Pedicure tool**

(30) Priority: 20.05.2002 IT VI20020100
(71) Applicant: Palmieri, Giuseppe, 36100 Vicenza (IT); Valerio, Andrea, 36010 Cavazzale di Monticello Conte Otto (Vicenza) (IT); Valerio, Fabio, 36010 Cavazzale di Monticello Conte Otto (Vicenza) (IT)
(72) Inventor: Palmieri, Giuseppe, 36100 Vicenza (IT); Valerio, Andrea, 36010 Cavazzale di Monticello Conte Otto (Vicenza) (IT); Valerio, Fabio, 36010 Cavazzale di Monticello Conte Otto (Vicenza) (IT)
(74) Representative: Bettello, Pietro, Dott. Ing.

(57) **Abstract**

The finding concerns a pedicure tool of the type which has a rectilinear handle (1), at one of the ends of which there is an interchangeable cutting tool (2). The fundamental characteristic of the device according to the finding consists of the fact that the tool is placed in a cartridge equipped with engagement means with corresponding means present at the end of the handle to determine the reversible attachment of the cartridge to the aforementioned handle.

## Description

The present finding concerns a pedicure tool, according to the general part of claim 1.

As is known, pedicure tools almost always have a rectilinear handle, at one of the ends of which there is an interchangeable cutting tool. Examples of tools of such a type are described in patent application for a utility model no. 64279/B/81, in which it is foreseen that a rectilinear-shaped handle is equipped, at one end thereof, with a conical clamp, with one fixed and one mobile jaw. An interchangeable cutting blade is arranged between said jaws. The closing of the clamp is obtained through the screwing of a threaded pin integral with the mobile jaw. In fact, such a device has the undoubted advantage, with respect to similar devices known previously, of allowing extremely easy replacement of the cutting blade, in particular when this blade, after use, is no longer suitable for being used. An improvement of such a device is described in patent application for a utility model no. VI93U000064, which has a constructive structure totally similar to that of the tool described previously, but where the apexes of the cutting portion of the blade are rounded, so that it is able to largely reduce the risk of injuring the person subjected the pedicure treatment.

In fact, much progression has been made in this technical field with respect to the utility models quoted previously. As an example, in any case, we quote European publication no. 685205, which concerns a multi-purpose pedicure instrument, which however is of the disposable single-use type and thus its use is intrinsically expensive, as well as clearly not adhering to environmental criteria.

The purpose of the present finding is that of realising a pedicure tool, the use of which is both more practical with respect to that of the models previously illustrated and described, as well as intrinsically cost-effective like the above.

This is obtained, according to the finding, by foreseeing that the interchangeable cutting tool, present at one of the ends of the rectilinear handle, be placed in a cartridge equipped with means intended for engaging with corresponding means present at the end of the handle, this being suitable for determining the reversible attachment of the cartridge to the handle itself. In practice, through the device according to the finding, a constructive arrangement is obtained which is comparable to that which is commonly found in domestic razors, where, in most cases, the blade or blades used for cutting facial hair are housed in a cartridge which can be clipped onto the head of the razor and can be replaced with another identical cartridge when the blade no longer cuts effectively.

These and other characteristics of the finding shall now be described in detail, with reference to a particular embodiment thereof, given as a non-limiting example, with the help of the attached table of drawings, where:
- figure 1 illustrates an overall view of the tool according to the model;
- figure 2 illustrates an enlarged view of the aforementioned tool with the cutting tool separated from its support handle.

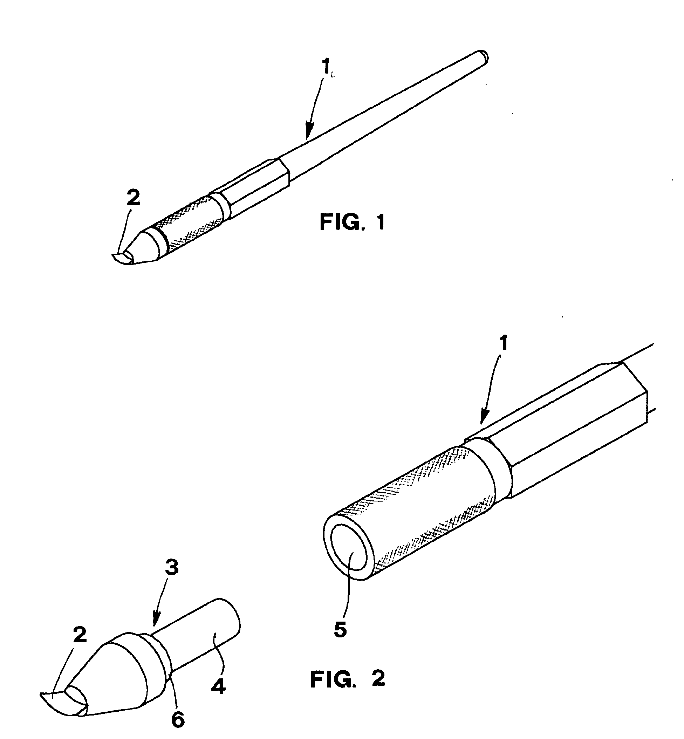

In figure 1 it can be seen that the device according to the finding consists of a pedicure tool, which has a rectilinear handle 1, at one of the ends of which there is an interchangeable cutting tool 2.

As can be seen in particular in figure 2, the novelty characteristic of the device according to the finding consists of the fact that the tool is arranged in a cartridge 3 equipped, in this particular embodiment, with a rectilinear projection 4, which can be inserted in a corresponding seat 5 present at the end of the handle 1. The projection 4 is equipped with an elastic portion 6 with a wider diameter, the outer surface of which is suitable for engaging by interference with the inner surface of the seat 5, determining the reversible locking of the cartridge with respect to the handle. However, it should be noted that any other system suitable for allowing the reversible engagement of the cartridge with respect to the end of the handle can be foreseen. In particular screw, bayonet, joint and similar systems can be foreseen, without for this reason departing from the scope of the patent.

From the above it can thus be seen how by using the device according to the finding, the user, who generally consists of the operator who carries out the pedicure operation on the client, has an article available to him which is intrinsically simple from the constructive point of view and thus cost-effective and in which he/she can without any problem take care of changing the cutting tool 2 when it is no longer able to effectively carry out its purpose. In this way he/she is able to replace the cutting tool with extreme ease and, above all, there is no risk of injuring oneself during the replacement step of the aforementioned cutting tool.

Moreover, the device according to the finding is suitable, thanks to its particular constructive and functional details, for being placed in a sterile package, from where it shall be removed only at the time of use. In this way, the device according to the finding is suitable for being inserted in the class of goods of "medical devices", particularly suitable for being used by professional qualified chiropodists.

## Claims

1. PEDICURE TOOL, of the type which has a rectilinear handle (1), at one of the ends of which there is an interchangeable cutting tool, said tool being **characterised in that** the tool (2) is placed in a cartridge (3) equipped with engagement means with corresponding means present at the end of the handle (1) for the reversible attachment of the cartridge to the handle.

2. PEDICURE TOOL, according to claim 1, **characterised in that** the cartridge (3) has a rectilinear projection (4), which can be inserted in a corresponding seat (5) present at the ends of the handle (1), the cylindrical seat being equipped with a portion (6) with increased diameter, made from elastic material and suitable for engaging by interference with the inner surface of the cylindrical seat (5).

3. PEDICURE TOOL, according to claim 1, **characterised in that** the engagement between the cartridge (3) and the handle (1) takes place through a bayonet attachment.

4. PEDICURE TOOL, according to claim 1, **characterised in that** the engagement between the cartridge (3) and the handle (1) takes place through a screw attachment means.

5. PEDICURE TOOL, according to claim 1, **characterised in that** the engagement between the cartridge (3) and the handle (1) takes place through jointing attachment means.
